# EUROPÄISCHE PATENTANMELDUNG

(11) **EP 4 613 212 A1**
(43) Veröffentlichungstag der Anmeldung: **10.09.2025**
(21) Anmeldenummer: 24162079.8
(22) Anmeldetag: 07.03.2024
(51) Int. Cl.: A61B 10/02, A61B 10/04, A61B 17/3203

(54) **VORRICHTUNG UND VERFAHREN ZUR OBERFLÄCHLICHEN ZELLGEWINNUNG**

(71) Anmelder: Erbe Elektromedizin GmbH, 72072 Tübingen (DE)
(72) Erfinder: FECH, Andreas, 72072 Tuebingen (DE); STRAUB, Frank, 72770 Reutlingen (DE); LINZENBOLD, Walter, 71034 Böblingen (DE); ENDERLE, Markus, 72070 Tuebingen (DE)
(74) Vertreter: Rüger Abel Patentanwälte PartGmbB

(57) **Zusammenfassung**

Die erfindungsgemäße Vorrichtung (15) zur Zellgewinnung ist ein Zusatzelement für ein Endoskop und wird somit von einem Endoskop geführt. Die Vorrichtung enthält ein Mittel zur Erzeugung eines Strahls (35), der die zu untersuchende Gewebeoberfläche unter einem spitzen Winkel (β) trifft, ohne sie zu verletzen. Der Strahl ist zum Beispiel ein Fächerstrahl oder ein Kegelstrahl. Die Vorrichtung kommuniziert mit einem Absaugkanal des Endoskops, über das während der Zellgewinnung Gas abgesaugt wird, um von dem Gewebe abprallende und von diesem aufgewirbelte Flüssigkeitströpfchen aufzunehmen und zusammen mit einem Luftstrom einer Sammeleinrichtung zuzuführen. So wird die Ansammlung verschiedener Gewebeproben im zu untersuchenden Organ und die somit möglicherweise zu Fehlzuordnungen führende Verschleppung von Gewebeproben vermieden.

## Beschreibung

Die Erfindung betrifft eine Vorrichtung, eine Einrichtung sowie ein Verfahren zur Zellgewinnung. Insbesondere können die Vorrichtungen und das Verfahren an Gewebeoberflächen, insbesondere Schleimhäuten, zum Beispiel im Magen, Darm, der Speiseröhre, im Gallenkanal oder an anderen insbesondere inneren Gewebeoberflächen eines tierischen oder menschlichen Patienten eingesetzt werden. Die Vorrichtung und das Verfahren eignen sich nicht nur zur Zellgewinnung, sondern auch zur Gewebebehandlung oder Gewebereinigung.

Ein wichtiger Anwendungsfall der Zellgewinnung ist die Untersuchung der Schleimhaut des Verdauungstrakts, um eventuelle Veränderungen frühzeitig zu diagnostizieren.. Dabei ist es häufig wünschenswert, die Gewebeoberfläche großflächig gerastert zu beproben und an den einzelnen Rasterpunkten aufgenommenen Gewebeproben nicht mit anderen Gewebeproben zu kontaminieren, um etwaige entdeckte Krebsvorstufen dem Entnahmeort sicher zuordnen zu können.

Es ist bekannt, Gewebeproben mittels eines Flüssigkeitsstrahls in einen Absaugkanal zu fördern, um diese einer Untersuchung zuzuführen. Entsprechende Instrumente sind aus der EP 4 072 448 A1 der EP 1 182 974 B1 der EP 2 019 628 A1**,** der EP 1 433 423 A1**,** der US 6,572,578 B1 und der EP 3 500 192 A1 bekannt. Diese Instrumente weisen jeweils einen Flüssigkeitskanal auf, der an einer Auslass-Öffnung einen Fluidstrahl erzeugt, der direkt auf die Saugöffnung eines Absaugkanals gerichtet ist.

Es sind auch Instrumente bekannt, bei denen ein Fluidstrahl direkt auf Gewebe geleitet wird, so zum Beispiel aus der EP 2 303 156 A2**,** der US 2021/0308484 A1 und der US 6,030,399 A**.** Letztgenannte Druckschrift dient der Entnahme von Blutproben aus der Haut.

Das aus der US 6,030,399 A bekannte Instrument weist einen Kopf auf, der einen Innenraum umschließt. Der Innenraum ist zu der Oberfläche der Haut hin offen, aus der Blut zu entnehmen ist. Der Rand der Öffnung ist auf die Haut aufzusetzen, wodurch der Innenraum nach außen hin abgedichtet wird. Um die Abdichtung möglichst vollständig sicherzustellen, ist der Rand der Öffnung mit einer entsprechenden Dichtung versehen. Ein Speisekanal dient zur Zuführung von Flüssigkeit in den Innenraum, wobei die Flüssigkeit als scharfer Strahl auf die Haut auftrifft und in diese einsticht. Auf diese Weise freigesetztes Blut oder Gemisch aus Blut und zugeführter Flüssigkeit wird über Absaugkanäle abgeführt, die mit dem Innenraum verbunden sind. Aus dem Fachartikel LightdaleCJ, Tiscornia-Wasserman P, Sethi A et al: "Endoscopy-Guided High-Pressure Spray "Power-Wash" to obtain Cytopathology For Detection Of Gastric Intestinal Metaplasia: A Proof Of Concept Study"; Gastrointest Endosc 2022; 95: AB457-AB458 ist bekannt, dass sich mit einem Strahl Zellen von einer Schleimhaut des Gastrointestinaltraktes abwaschen lassen.

Bei der Beprobung von Gewebeoberflächen auf Metaplasien soll möglichst unblutig vorgegangen werden. Es geht darum, lediglich Zellen zu entnehmen, ohne die Gewebeoberfläche zu schädigen.

Davon ausgehend ist es Aufgabe der Erfindung, eine Vorrichtung und eine Einrichtung zur unblutigen Zellgewinnung zu schaffen. Außerdem ist es Aufgabe der Erfindung, ein entsprechendes Zellgewinnungsverfahren anzugeben.

Diese Aufgaben werden mit der Vorrichtung nach Anspruch 1, einer Einrichtung nach Anspruch 14 und dem Verfahren nach Anspruch 15 gelöst:

Die erfindungsgemäße Vorrichtung dient zur Zellgewinnung und kann somit zur oberflächlichen Gewebebeprobung, aber auch zur Gewebereinigung oder Gewebebehandlung eingesetzt werden. Insbesondere ist sie für die Beprobung von Schleimhäuten, zum Beispiel der Magenschleimhaut, vorgesehen. Das Arbeitsprinzip der erfindungsgemäßen Vorrichtung beruht darauf, eine Gewebeoberfläche mit einem aus Strahl zu beaufschlagen und von der Gewebefläche zurückgeworfene oder abgelöstes Material über einen Absaugkanal vorzugsweise zusammen mit einem Luft- oder Gasstrahl abzusaugen und in einer Sammeleinrichtung zu sammeln. Der Strahl kann die Düse in einer Ausführungsform als nicht in Tröpfchen zerfallener Vollstrahl oder in einer anderen Ausführungsform als aus Tröpfchen bestehender Strahl verlassen und auf das Gewebe gelangen. Vorzugsweise weitet sich der Strahl mit zunehmendem Abstand von der Düse auf.

Die erfindungsgemäße, zur Probenentnahme dienende Vorrichtung weist einen Kopf auf, der einen Innenraum umschließt. An dem Kopf ist eine Öffnung ausgebildet, die den Innenraum mit der Umgebung verbindet und deren Rand auf die Gewebeoberfläche aufsetzbar ist. Der Rand legt eine Mündungsebene fest, die eine gedachte mathematische an dem Rand der Öffnung anliegende Ebene ist. Innerhalb des Kopfs ist eine Einrichtung zur Erzeugung eines Strahls vorgesehen, der in einem von Null verschiedenen spitzen Winkel zur Flächennormalen der Mündungsebene steht. Damit ist der Strahl so gerichtet, dass er eine an der Öffnung anliegende Gewebeoberfläche streifend trifft.

Zur Erzeugung des Strahls ist ein Speisekanal vorgesehen, der an einer Düse endet. Der Speisekanal ist an eine Flüssigkeitsfördereinrichtung, zum Beispiel eine Pumpe, angeschlossen oder anschließbar, sodass der Düse Flüssigkeit unter Druck zugeleitet wird und aus der Düse ein Flüssigkeitsstrahl austritt. Die Düse kann eine Sprühdüse sein, die unmittelbar einen aus Tröpfchen bestehenden Strahl erzeugt. Die Düse kann auch dazu eingerichtet sein, einen scharfen (z.B. linienförmigen) Strahl zu erzeugen, der auf eine in dem Kopf angeordnete Prallfläche gerichtet ist. Die Prallfläche kann dazu eingerichtet sein, den Strahl umzulenken, um einen linienförmigen, fächerförmigen, kegelförmigen oder anderweitig geformten Strahl zu erzeugen und in Richtung der Öffnung zu leiten. Ein fächerförmiger vorzugsweise nicht in Einzeltropfen zerfallener Strahl wird dabei vorzugsweise so auf die Mündungsebene hin gerichtet, dass der er in einer Fächerebene liegt, deren Normale in einer Axialebene liegt, die mit der Mündungsebene einen rechten Winkel einschließt. Die Genauigkeit dieser Angaben ist mit einer Toleranz von ± 20°, vorzugsweise ± 10° zu verstehen.

Der die Düse verlassende Strahl kann ein Vollstrahl aus unzerstäubter Flüssigkeit sein. Die Düse und/oder die Prallfläche können auch dazu eingerichtet sein, die aus der Düse austretende Flüssigkeit zu zerstäuben, um einen aus Tröpfchen bestehenden Strahl zu erzeugen. Die Prallfläche ist dazu vorzugsweise schräg zu dem aus der Düse austretenden Strahl gerichtet. Sie kann an der dem Strahl zugewandten Seite eben ausgebildet sein oder eine Profilierung aufweisen, die die Zerstäubung des Strahls fördert. Mit dem Strahl werden Zellen einer Gewebeoberfläche, z.B. Epithelzellen einer Schleimhaut, ohne Abtrag tieferer Gewebeschichten von der Gewebeoberfläche abgewaschen. Es werden insbesondere keine Zellen aus der Tiefe (d.h. tiefen Schichten) des Gewebekörpers entnommen.

Zu der Vorrichtung gehört eine Belüftungseinrichtung, die dazu eingerichtet ist, Gas oder Luft aus der Umgebung in den Innenraum des Kopfs gelangen zu lassen. Der so erzeugte Luft- oder Gasstrom soll die mit dem Strahl auf die Gewebeoberfläche aufgebrachte Flüssigkeit insbesondere aus schwer oder nicht zugänglichen Bereichen, wie Toträumen und Hinterschneidungen, möglichst vollständig aufnehmen und zu der Sammeleinrichtung tragen. Der Gasstrom soll dabei so niedrig wie möglich sein, um eine stabile Insufflation des (Organ-)Lumens zu gewährleisten. Bei einem zu hohen Gasstrom wird das Organ desuffliert und die Sicht geht verloren. Die Belüftungseinrichtung soll deswegen kleine, den Gasstrom begrenzende Öffnungen aufweisen. Die Stelle der Gewebeoberfläche, von der die Gewebeprobe entnommen worden ist, ist dadurch nach der Probenentnahme weitgehend trocken, so dass eine Verschleppung von Epithelzellen auf andere Stellen der Gewebeoberfläche oder Flüssigkeitsansammlungen vermieden wird.

Mit dem Innenraum ist ein Absaugkanal verbunden, der zu einer Absaugeinrichtung, zum Beispiel einer Saugpumpe, führt. Durch die Belüftungseinrichtung kann während der Zellgewinnung Luft oder Gas in den Innenraum des Kopfs nachströmen und somit einen in den Absaugkanal führenden Luftstrom bilden. Dieser nimmt die von der Gewebeoberfläche reflektierten Flüssigkeitströpfchen und die von den Tröpfchen aufgenommenen Zellen oder sonstigen Partikel auf und führt sie über den Absaugkanal zu der Sammeleinrichtung. Vorzugsweise ist die Belüftungseinrichtung so ausgebildet, dass im Bereich der Öffnung des Kopfs eine solche Strömungsgeschwindigkeit des einströmenden Gases erreicht wird, die dazu ausreicht, ein Austreten und Ablaufen der Flüssigkeit von dem Kopf weg zu verhindern. Das einströmende Gas soll die Flüssigkeit von dem Gewebe in den Absaugkanal mitnehmen. Damit ist die eindeutige Zuordnung gewonnener Proben zu den jeweiligen Probenentnahmestellen möglich.

Vorzugsweise sind die Strahlrichtung des Strahls und die Strömungsrichtung der in die Öffnung einströmenden Luft (oder Gases) an der Öffnung einander entgegengesetzt gerichtet. Währende der Strahl in flachem Winkel zu der Gewebeoberfläche hin gerichtet ist, ist die Strömungsrichtung der seitlich zuströmenden Luft von der Gewebeoberfläche weg gerichtet. Um dies zu erreichen ist der sich unmittelbar an die Öffnung des Kopfs anschließende Teil des Absaugkanals in einem Winkel zu dem Strahl orientiert, der kleiner als 180° ist. Während die Richtung des Strahls eine Komponente zu der Gewebeoberfläche hin aufweist, weist die Richtung des Gas- oder Luftstroms eine Komponente auf, die von der Gewebeoberfläche weg gerichtet ist. Dazu sind in dem Kopf entsprechende Strömungsleitmittel, z.B. in Gestalt der Düse, der Prallfläche und des Absaugkanals vorgesehen. So wird eine Verschleppung von Proben an andere Orte der Gewebeoberfläche vermieden.

Die erfindungsgemäße Vorrichtung eignet sich insbesondere zur lokalen Beprobung einer Gewebeoberfläche zum Beispiel in einem Raster. Die an einer Beprobungsstelle durch Besprühen der Oberfläche des Gewebes gelösten einzelnen Zellen und die dazu eingesetzte Flüssigkeitsmenge reicht nicht, um den Absaugkanal zu füllen. Durch den Luft- oder Gasstrom, der sich von der Belüftungseinrichtung ausgehend in den Absaugkanal erstreckt, gelingt es dennoch, die an der Probenentnahmestelle aufgenommenen Tröpfchen und Partikel weitgehend restlos zu einem Probensammelgefäß zu fördern. Auf diese Weise wird eine Vermischung von an verschiedenen Stellen aufgenommenen Proben miteinander wirksam ausgeschlossen.

Als Belüftungseinrichtung können eine oder mehrere von der Umgebung in den Innenraum des Kopfs führenden Öffnungen dienen. Zusätzlich oder alternativ können Mittelvorgesehen sein, die einen dichten Abschluss des Rands der Öffnung des Kopfs auf dem der Gewebeoberfläche verhindern. Zum Beispiel kann die Öffnung an dem Kopf so angeordnet sein, dass sie in Gebrauch nicht oder nur über einen Teil ihres Randes an der Gewebeoberfläche anliegt. An dem Kopf kann auch ein Vorsprung angeordnet sein, der sich in Gebrauch auf dem Gewebe abstützt und dadurch die Öffnung in Distanz zu der Gewebeoberfläche hält.

Die erfindungsgemäße Vorrichtung kann einen rohrförmigen Schaft aufweisen, der dazu eingerichtet ist, in den Arbeitskanal eines Endoskops eingesteckt oder auf das Ende eines Endoskops aufgesteckt zu werden. Die Vorrichtung und das Endoskop zusammen bilden somit eine Einrichtung, mit der die stellenweise Beprobung von Gewebeoberflächen möglich ist, ohne die Gewebeoberfläche zu verletzen. Dabei hat es sich insbesondere als zweckmäßig herausgestellt, wenn der Schaft der Vorrichtung flexibel ausgebildet ist, sodass ihr Kopf zur Längsrichtung des Endoskops radial etwas nachgiebig gehalten ist. Damit gelingt es, eine Gewebeoberfläche in einem Raster an sehr vielen Stellen zu beproben, wobei der Kopf der Vorrichtung jeweils mit einer Vorspannung an der Gewebeoberfläche anliegen kann oder das Gewebe durch Unterdruck an die Öffnung herangezogen wird.

Zu der Vorrichtung kann insbesondere ein Instrument oder ein Schlauch gehören, der lang genug ist, um durch den Arbeitskanal des Endoskops von einer Flüssigkeitsquelle zu der Vorrichtung geführt zu werden. Die Vorrichtung weist vorzugsweise einen Sitz auf, in dem der Schlauch oder das Instrument gefasst sind. Das Instrument oder der Schlauch weist an seinem Ende vorzugsweise eine Düse auf, die zur Erzeugung eines Flüssigkeitsstrahls eingerichtet ist. Vorzugsweise ist die Düse derart ausgebildet, dass sie den Strahl in Axialrichtung austreten lässt. Die Axialrichtung ist dabei die Längsrichtung des Schlauchs oder schlauchartigen Instruments, die mit der Axialrichtung des zylindrischen Schafts des Instruments übereinstimmt. In diesem Fall ist die Prallfläche der Düse gegenüberliegend angeordnet und dazu eingerichtet ist, den Strahl umzulenken und ihm eine gewünschte Form, vorzugsweise Fächerform zu geben. Bei der Erzeugung eines Fächerstrahls kann die auf die Platte treffende Flüssigkeit zunächst an der Oberfläche der Platte entlang fließen. An der Kante der Platte, an der die Flüssigkeit die Platte verlässt kann dann die Fächerform entstehen. Vor dem Auftreffen auf die Platte kann der Strahl beispielsweise kegelförmig sein. Als Alternative ohne Verwendung einer Platte kommt ebenfalls ein Kegelstrahl in Frage.

Die Hauptachse des Strahls ist vorzugsweise schräg zu der Öffnung des Kopfs und somit auch schräg zu der Oberfläche des zu behandelnden Gewebes gerichtet. Vorzugsweise trifft der Strahl unter einem Winkel auf das Gewebe auf, der kleiner als 60° ist. Dies verhindert ein tiefes Eindringen des Strahls in das Gewebe, was Gewebeverletzungen verhindert oder ausschließt.

Die Prallfläche kann eben oder profiliert ausgebildet sein, um eine gewünschte Strahlform zum Beispiel einen Fächerstrahl oder einen Strahl mit gewünschtem Strahlquerschnitt zu erzeugen.

Bei dem erfindungsgemäßen Verfahren wird ein Strahl aus Flüssigkeit vorzugsweise unter einem spitzen Winkel auf eine Gewebeoberfläche gerichtet, um einige Zellen von der Oberfläche abzustreifen. Vorzugsweise ist der Winkel kleiner als 60°. Die sich von der Gewebeoberfläche lösenden Flüssigkeitstropfen werden vorzugsweise von einem Gas- oder Luftfluss aufgenommen, eingesammelt und einer Sammeleinheit zugeführt. In dieser können verschieden Gefäße oder Abteilungen vorgesehen sein, um Proben verschiedener Probeentnahmestellen voneinander getrennt zu halten. Damit ist eine Zuordnung verschiedener Proben zu verschiedenen Probeentnahmestellen möglich. Die mögliche vollständige Flüssigkeitsabfuhr von jeder Probeentnahmestelle mittels ausreichend starken Luft- oder Gasstroms macht eine spätere Zuordnung von Gewebeproben zu Gewebeentnahmestellen ohne weiteres möglich.

Weitere vorteilhafte Einzelheiten von Ausführungsformen der Erfindung ergeben sich aus der Beschreibung, der Zeichnung oder Ansprüchen. In der Zeichnung zeigen:
Figur 1 ein in einen Patienten eingeführtes Endoskop mit erfindungsgemäßer Vorrichtung zur Gewebeprobenentnahme, in schematisierter Darstellung,
Figur 2 das distale Ende des Endoskops und die erfindungsgemäße Vorrichtung, in längsgeschnittener schematisierter Darstellung,
Figur 3 bis 5 alternative Ausführungsformen von Vorrichtungen zur Verwendung an Endoskopen,
Figur 6 eine Prinzipdarstellung mit im Längsschnitt, zur Veranschaulichung des Prinzips der Arbeit der erfindungsgemäßen Vorrichtung, und
Figur 7 eine Prinzipskizze der erfindungsgemäßen Vorrichtung zur Veranschaulichung ihrer geometrischen Verhältnisse.

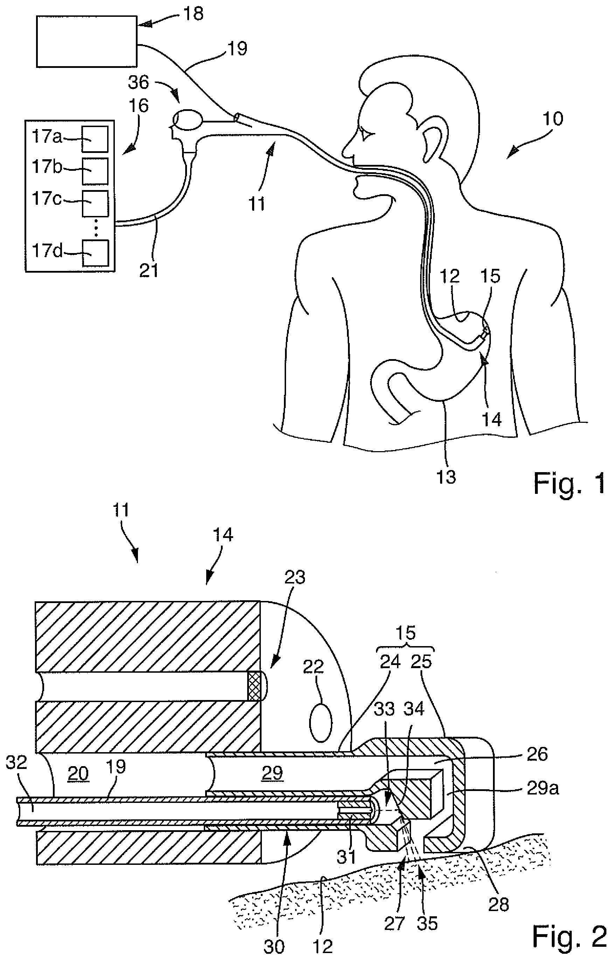

In Figur 1 ist die Untersuchung eines Patienten 10 mittels eines Endoskops 11 veranschaulicht, das hier zur Beprobung der Schleimhaut 12 seines Magens 13 genutzt wird. Das in einem Winkelbereich bewegliche distale Ende 14 des Endoskops 11 wird dabei zur Positionierung einer daran befestigten Vorrichtung 15 genutzt, die zur punktweisen oder stellenweisen Probenentnahme genutzt wird. Unter "punktweise" wird dabei die Probenentnahme auf einer kleinen, wenige Quadratmillimeter umfassenden, abgegrenzten Fläche verstanden. Bei einer "stellenweisen" Probenentnahme kann die Fläche auch größer sei. Die einzelnen Probeentnahmestellen können in einem Raster über die Magenschleimhaut 12 verteilt sein, so dass insgesamt mittels vieler Proben ein schnelles Screening einer großen Fläche möglich ist.

Die entnommenen Gewebeproben können dann in einer Sammeleinrichtung 16 gesammelt werden. Die Sammeleinrichtung kann eine Saugvorrichtung und eine Abscheideeinrichtung enthalten, um die Flüssigkeit von dem Luft- oder Gasstrom abzuscheiden, der aus dem Endoskop kommt. Vorzugsweise weist die Sammeleinrichtung 16 für jede Probeentnahmestelle ein gesondertes Sammelgefäß 17a, 17b, 17c, 17d auf. Die Sammelgefäße 17 können nach der Probenentnahme in ein Analyselabor oder zu einer Analysevorrichtung verbracht werden, um die darin enthaltene Flüssigkeit nach medizinischen Gesichtspunkten zu untersuchen. Es kann aber auch für alle Proben ein gemeinsames Sammelgefäß vorgesehen sein.

Das Endoskop 11 ist nicht nur an die Sammeleinrichtung 16, sondern außerdem an eine Flüssigkeitsversorgungseinrichtung 18 angeschlossen, die unter Druck stehende Flüssigkeit, beispielsweise physiologische Kochsalzlösung, liefert. An die Flüssigkeitsversorgungeinrichtung 18 ist ein schlauchartiges Instrument 19 angeschlossen, mittels dessen der Vorrichtung 15 unter Druck stehende Flüssigkeit zugeführt wird.

Figur 2 veranschaulicht das distale Ende 14 des Endoskops 11 und die daran befestigte Vorrichtung 15 in längsgeschnittener schematischer Darstellung. Das Endoskop 11 weist mindestens einen Arbeitskanal 20 auf, der, wie Figur 1 zeigt, über einen Schlauch 21 an die Sammeleinrichtung 16 angeschlossen ist. Die Sammeleinrichtung 16 ist dabei dazu eingerichtet, einen Unterdruck, d.h. einen Sog zu erzeugen, um über den Arbeitskanal 20 Gase und Luft aus dem Magen 13 abzusaugen. Die entsprechenden zur Sog- oder Unterdruckerzeugung vorgesehenen Mittel sind in Figur 1 nicht veranschaulicht. Ebenso sind die Mittel zum Trennen der von dem Luftstrom mitgeführten Flüssigkeit und zur Zuführung derselben in die Sammelgefäße 17 nicht veranschaulicht.

Das Endoskop kann außer dem Arbeitskanal 20 einen weiteren Kanal 22 aufweisen, der dazu genutzt werden kann, dem Magen 13 Gas oder Luft zuzuführen. Außerdem kann das Endoskop eine optische Beobachtungseinrichtung 23 aufweisen, um dem Behandler Sicht auf die Vorrichtung 15 und die darunter und darum befindliche Magenschleimhaut 12 zu gewähren.

Die Vorrichtung 15 weist in den in den Figuren 2 bis 4 veranschaulichten Ausführungsformen jeweils einen Schaft 24 auf, der in den Arbeitskanal 20 des Endoskops 11 einsteckbar ist. Der Schaft 24 trägt einen Kopf 25, der einen Innenraum 26 umschließt und somit ein Gehäuse bildet. Der Kopf 25 und der Schaft 24 können einteilig, d.h. naht- und fugenstellenlos aus ein und demselben Material, zum Beispiel Kunststoff, ausgebildet sein. Vorzugsweise ist der Schaft 24 dabei flexibel, sodass der Kopf 25 unter der Vorspannung des federnden Schafts 24 an der Gewebeoberfläche 12 entlanggeführt werden kann und dabei an dieser anliegt.

An der der Gewebeoberfläche 12 zugewandten Seite weist der Kopf 25 eine Öffnung 27 auf, die zur Probenentnahme dient. In unmittelbarer Nachbarschaft der Öffnung 27 kann der Kopf 25 einen Vorsprung 28 aufweisen, der einen Abstandshalter bildet und bei Nutzung der Vorrichtung 15 an dem Gewebe anliegen kann.

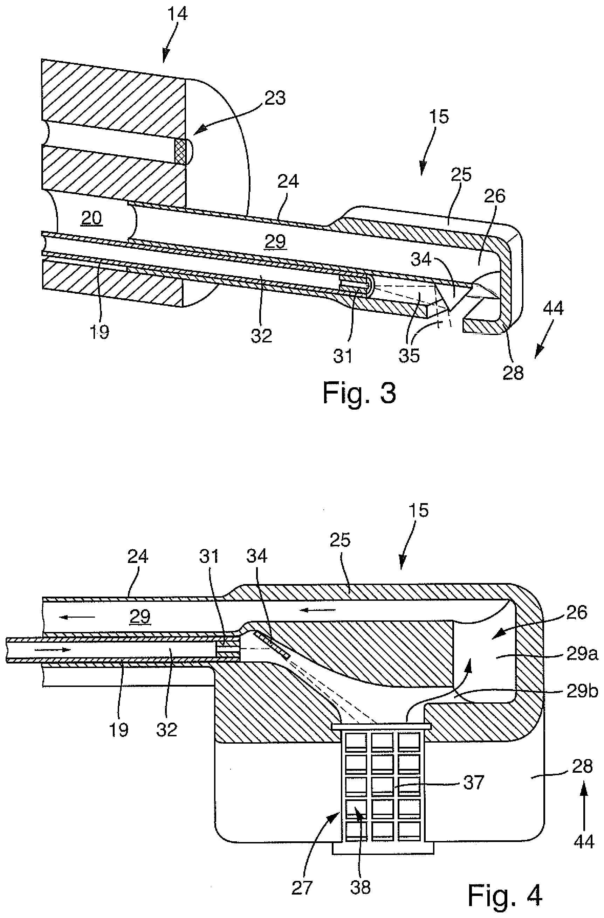

Von dem Innenraum 26 führt ein Absaugkanal 29 durch den Schaft 24 und mündet in den Arbeitskanal 20. Der Absaugkanal 29 weist einen Abschnitt 29a (in Figur 4 auch 29b) auf, der Luft von der Öffnung 27 weg führend ausgerichtet ist.

Die Vorrichtung 15 weist außerdem einen Sitz 30 für das distale Ende des Instruments 19 auf. Der Sitz 30 kann durch einen sich parallel zu dem Absaugkanal 29 durch den Schaft 24 erstreckenden Durchgang gebildet sein, in dem das Instrument 19 reibschlüssig sitzt. Es kann dabei gegen einen in Figur 2 nicht weiter veranschaulichten Vorsprung bzw. Verengung des Sitzes 30 anliegend gehalten sein. Der Schaft 24 wiederum sitzt vorzugsweise reibschlüssig in dem Arbeitskanal 20.

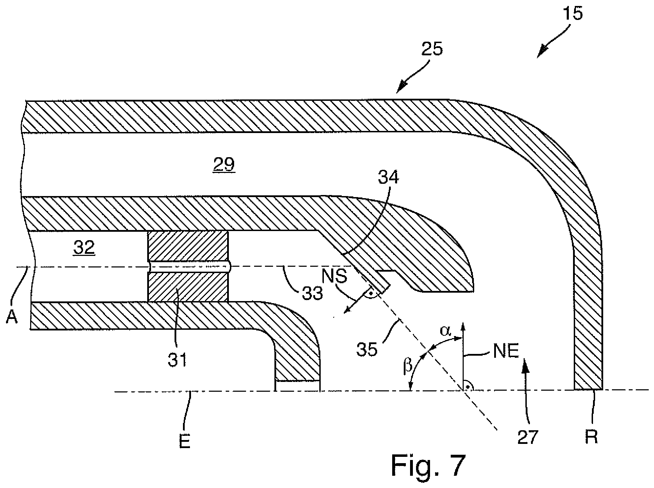

An dem distalen Ende des Instruments 19 ist vorzugsweise eine Düse 31 angeordnet, die durch das Lumen 32 des Instruments 19 herangeführte Flüssigkeit als Strahl z.B. als Linienstrahl oder Kegelstrahl austreten lässt. In Figur 2 und Figur 7 ist ein solcher Strahl 33 punktiert veranschaulicht. Der Düse 31 bzw. der Mündung ihres Düsenkanals gegenüberliegend ist in dem Innenraum des Kopfs 25 eine Prallfläche 34 angeordnet, gegen die der Strahl 33 gerichtet ist. Die ebene oder profilierte Prallfläche 34 erzeugt einen Strahl 35, der die Öffnung 27 durchquert und in einem spitzen Winkel auf die Gewebeoberfläche 12 trifft. Der Strahl 35 ist beispielsweise ein Fächerstrahl, der ein zusammenhängendes Flüssigkeitsblatt bildet oder in einzelne Tröpfchen aufgelöst ist. Der Kopf 25 hält die Düse 31 und die Prallfläche 34 in einem Abstand zu der Gewebeoberfläche 12, so dass der erzeugte Fächerstrahl 35 einen nicht nur punktförmigen sondern größeren Bereich der Gewebeoberfläche 12 innerhalb der Öffnung 27 trifft. Der Fächerstrahl kann z.B. einen großen Teil der Öffnung 27 oder die gesamte Breite der Öffnung 27 einnehmen. So wird eine großflächige Biopsie ermöglicht.

Die geometrischen Verhältnisse sind in Figur 7 veranschaulicht. Der längs der Längsachse A der Düse 31 austretende Strahl 33 ist ein Linearstrahl oder ein Kegelstrahl. Er trifft auf die Prallfläche 34 und verlässt diese in einem stumpfen Winkel zu seiner ursprünglichen Richtung A als Fächerstrahl 35. Dabei schneidet er eine gedachte, an den Rand 27a der Öffnung angelegte Ebene E. Der Strahl 35 schließt mit dem Normalenvektor NE der Fläche E einen spitzen, von Null verschiedenen Winkel α ein. Der fächerförmige Strahl 35 liegt dabei in einer Ebene, deren Normalenvektor NS mit der Längsachse A in einer gemeinsamen Ebene liegt, die die Ebene E entlang einer in Längsrichtung liegenden Linie rechtwinklig schneidet. Der Strahl 35 streift eine an die Öffnung 27 angelegte Gewebefläche unter dem spitzen Winkel β.

Wenn der Strahl 35 auf die Gewebeoberfläche 12 trifft, nimmt er dort einzelne Zellen oder Zellcluster auf. Die Flüssigkeit wird dann von dem Sog des der Öffnung 27 zuströmenden Gases mitgerissen und letztendlich der Sammeleinrichtung 16 zugeführt.

Will ein Behandler, beispielsweise ein Chirurg, einem Patienten 10 mehrere einzelne Proben von der Magenschleimhaut 12 entnehmen, um diese Proben einer Analyse zuzuführen, versieht er zunächst das Endoskop 11 mit der Vorrichtung 15, indem er das Instrument 19 durch den Schaft des Endoskops führt. Sobald das distale Ende des Instruments 19 aus dem Arbeitskanal 20 ragt, fädelt er das distale Ende des Instruments 19 in den Sitz 30 ein und steckt den Schaft 24 dann in den Arbeitskanal 20, wo er reibschlüssig (oder auf sonstige Weise) gehalten ist. Ein Teil des Schafts 24 sitzt in dem Arbeitskanal 20, während ein anderer Teil des Schafts 24 aus dem Arbeitskanal 20 herausragt und den Kopf 25 trägt.

Im nächsten Schritt wird das Endoskop 11 durch die Speiseröhre in den Magen 13 oder in ein anderes Hohlorgan eingeführt. Z.B. kann das Endoskop durch den Anus eines Patienten in seinen Darm eingeführt werden.

Durch die an dem proximalen Ende des Endoskops 11 vorhandenen Bedienelemente 36 kann der Behandler das distale Ende 14 des Endoskops 11 abwinkeln und in eine gewünschte Lage bringen, sodass die Vorrichtung 15 mit dem Vorsprung 28 an der Magenschleimhaut 12 anliegt. Zur Probenentnahme wird nun die Flüssigkeitsversorgungseinrichtung 18 aktiviert, wodurch die in dem Lumen 32 stehende Flüssigkeit nun als scharfer Strahl 33 aus der Düse 31 austritt. Auf die Prallfläche 34 auftreffend fächert sich der Strahl 33 zu dem Strahl 35 auf, der die Magenschleimhaut 12 trifft. Zugleich erzeugt die Sammeleinrichtung 16 einen Sog, wodurch über den Arbeitskanal 20 Luft oder Gas aus dem Innenraum 26 abgesaugt wird. An der Magenschleimhaut 12 entlang strömt nun Luft oder Gas aus dem Magen 13 durch die Öffnung 27 in den Innenraum 26 und reißt dabei von der Magenschleimhaut 12 abprallende Tröpfchen mit und wirbelt an der Magenschleimhaut 12 anhaftende Flüssigkeit auf. Von dem Strahl 35 gelöste Zellen werden zusammen mit der Flüssigkeit in den Innenraum 26 und über den Absaugkanal 29 und den Arbeitskanal 20 zu der Sammeleinrichtung 16 geführt. Die Sammeleinrichtung 16 weist Mittel, zum Beispiel Prallflächen, Zyklonabscheider oder andere Abscheidereinrichtungen auf, die die von dem Gasstrom getragenen Flüssigkeitspartikel abscheiden und in eines der Sammelgefäße 17a bis 17d leiten.

Soll an einer anderen Stelle der Magenschleimhaut 12 eine Probe genommen werden, wird zunächst die Flüssigkeitszufuhr für das Lumen 32 und danach die Gasabsaugung durch die Sammeleinrichtung 16 abgeschaltet. Der Behandler kann nun die Vorrichtung 15 an eine andere Stelle der Magenschleimhaut 15 führen und den eben beschriebenen Prozess wiederholen.

Dies kann beliebig oft geschehen, um die Magenschleimhaut 12 in einem gewünschten Raster zu untersuchen und an jeder Stelle des Rasters eine Probe zu nehmen. Die Proben können gesondert erfasst und somit gesondert untersucht werden, um später verdächtige Stellen der Magenschleimhaut 12 behandeln zu können.

Figur 3 veranschaulicht eine abgewandelte Ausführungsform der erfindungsgemäßen Vorrichtung 15, die sich durch einen besonders langen Schaft 24 auszeichnet. Außerdem kann sie, anders als zuvor beschrieben, eine Düse 31 aufweisen, die den Strahl 35 unmittelbar erzeugt. Dieser kann direkt auf die Gewebeoberfläche 12 treffen oder, wie es in Figur 3 dargestellt ist, mittels der Prallfläche 34 auf das Gewebe geleitet werden. Die Erzeugung des Strahls 35 bereits durch die Düse 31 kann genutzt werden, um eine besonders schonende Behandlung herbeizuführen. Im Übrigen gilt die vorige Beschreibung und im Zusammenhang mit den bereits eingeführten Bezugszeichen entsprechend.

Figur 4 veranschaulicht eine weitere leicht abgewandelte Ausführungsform der Vorrichtung 15. Bei dieser ist an der Öffnung 27 ein Gitter 37 angeordnet, das für einen definierten Abstand zwischen dem Strahl 35 und der Gewebeoberfläche 12 sorgt. Auch wird ein Einsaugen größerer Partikel in den Innenraum 26 und deren Abführung über den Absaugkanal 29 vermieden. Anstelle des relativ grobmaschigen Gitters 37 kann auch ein feinmaschigeres oder ein Sieb vorgesehen sein. Das Gitter 37 kann eine oder mehrere Erhebungen aufweisen, um ein Festsaugen der Öffnung 27 an der Gewebeoberfläche 12 und/oder ein Einsaugen derselben in den Kopf 15 zu verhindern. Alternativ kann der Kopf 26, wie schon vorstehend beschrieben, den Vorsprung 28 aufweisen, der als Abstandshalter zwischen dem Gewebe bzw. der Magenschleimhaut 12 und der Öffnung 27 dient, wie es in Figur 2 angedeutet war. Alternativ ist es auch möglich, an dem Kopf 25 zum Beispiel seitlich in Nachbarschaft der Öffnung 27 ein- oder beidseits Lufteinlassöffnungen 38 vorzusehen. In Figur 4 ist die Lufteinlassöffnung 38 teilweise durch das Gitter 37 verdeckt. Durch die Lufteinlassöffnung 38 strömt Gas oder Luft dem Sog der Sammeleinrichtung 16 folgend in den Innenraum 26 und trägt somit Zellproben enthaltende Flüssigkeitströpfchen zu der Sammeleinrichtung 16.

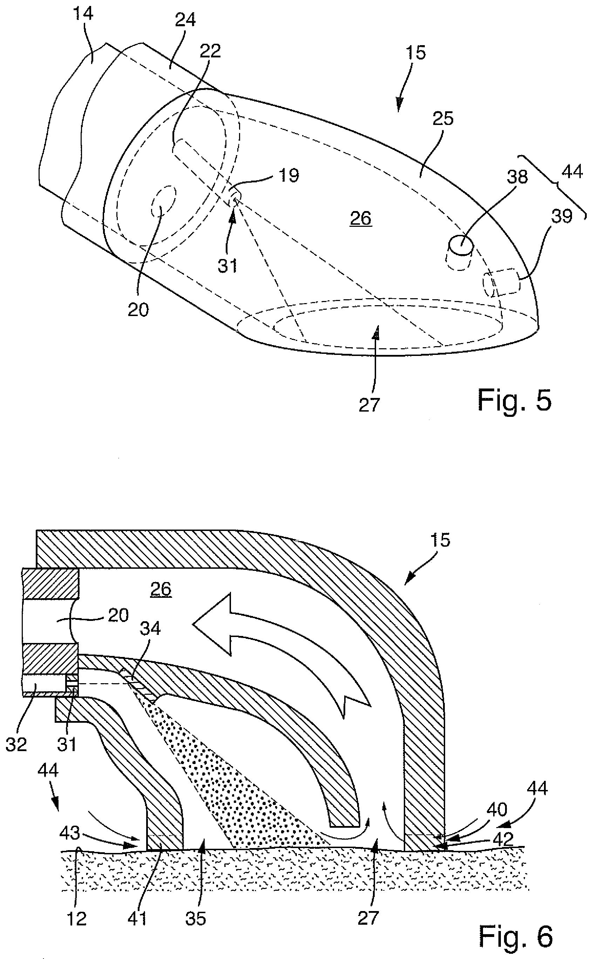

Die bisherigen Ausführungsformen der Vorrichtung 15 waren jeweils über ihren Schaft 24 in dem Arbeitskanal 20 befestigt. Es ist jedoch auch möglich, die Vorrichtung 15 auf das distale Ende 14 des Endoskops 11 aufzustecken. Solche Ausführungsformen gehen aus den Figuren 5 und 6 hervor.

Eine erste Variante der Vorrichtung 15 geht aus Figur 5 hervor, in der der Kopf 25 und der Schaft 24 durchsichtig dargestellt sind. Der Kopf 25 ist nach Art einer Haube ausgebildet, die an einer Seite die Öffnung 27 aufweist, die auf das Gewebe bzw. die Magenschleimhaut 12 aufzusetzen ist. Der Absaugkanal 20 kommuniziert mit dem Innenraum 26. Durch einen weiteren Arbeitskanal 22 erstreckt sich das Instrument 19, dessen distales Ende aus dem Arbeitskanal 22 herausragt. Um Luftzutritt zu dem Innenraum 26 zu gewähren, sind eine oder mehrere Luftzutrittsöffnungen 38, 39 vorgesehen, die eine Verbindung zwischen der Umgebung und dem Innenraum 26 durch die Wandung des Kopfs 25 hindurch herstellen.

Zur weiteren Verdeutlichung des Funktionsprinzips ist in Figur 6 eine Ausführungsform der Vorrichtung 15 veranschaulicht, bei der die Belüftungseinrichtung weder durch einen Vorsprung 28 noch durch in der Wandung des Kopfs vorgesehene Belüftungsöffnungen 38, 39, sondern durch gezielt am Rand der Öffnung 27 vorhandene Vorsprünge 40, 41 gebildet ist. Die Vorsprünge 40, 41 verhindern ein dichtes Anlegen der Magenschleimhaut 12 an den Rand der Öffnung 27. Durch die verbleibenden Abstände 42, 43 kann, wie durch Pfeile symbolisiert ist, nahezu ungehindert Luft in den Innenraum 26 einströmen, um von dem Strahl 35 herrührende Tröpfchen und mit Hautpartikel bzw. Zellen in den Absaugkanal 20 tragen.

Wie ersichtlich, können sowohl der Vorsprung 28 als auch die Öffnungen 38, 39 wie auch die Vorsprünge 40, 41 als Belüftungseinrichtung 44 dienen, um Luft oder ein anderes gasförmiges Medium aus der Umgebung in den Innenraum 26 und aus diesem in den Absaugkanal 20 strömen zu lassen.

Die erfindungsgemäße Vorrichtung 15 zur Zellgewinnung ist ein Zusatzelement für ein Endoskop 11 und wird somit von einem Endoskop 11 geführt. Die Vorrichtung 15 enthält ein Mittel zur Erzeugung eines Strahls 35, der die zu untersuchende Gewebeoberfläche 12 unter einem von Null verschiedenen spitzen Winkel β trifft, ohne sie zu verletzen. Der Strahl 35 ist zum Beispiel ein Fächerstrahl oder ein Kegelstrahl. Die Vorrichtung kommuniziert mit einem Absaugkanal des Endoskops 11, über das während der Zellgewinnung Gas abgesaugt wird, um von dem Gewebe abprallende und von diesem aufgewirbelte Flüssigkeitströpfchen aufzunehmen und zusammen mit einem Luftstrom einer Sammeleinrichtung 16 zuzuführen. So wird die Ansammlung verschiedener Gewebeproben im zu untersuchenden Organ und die somit möglicherweise zu Fehlzuordnungen führende Verschleppung von Gewebeproben vermieden.

### Bezugszeichen:

- 10: Patient
- 11: Endoskop
- 12: Magenschleimhaut
- 13: Magen
- 14: distales Ende des Endoskops
- 15: Vorrichtung
- 16: Sammeleinrichtung
- 17: Sammelgefäße
- 17a - 17d: einzelne Sammelgefäße
- 18: Flüssigkeitsversorgungseinrichtung
- 19: Instrument
- 20: Arbeitskanal
- 21: Schlauch
- 22: Kanal
- 23: optische Einrichtung
- 24: Schaft
- 25: Kopf
- 26: Innenraum
- 27: Öffnung
- R: Rand der Öffnung 27
- 28: Vorsprung / Abstandshalter
- 29: Absaugkanal
- 29a: Abschnitt des Absaugkanals
- 29b: weiterer Abschnitt des Absaugkanals
- 30: Sitz
- 31: Düse
- 32: Lumen des Instruments 11
- 33: Strahl
- 34: Prallfläche
- 35: Strahl
- A: Längsachse
- E: Ebene des Öffnungsrands R
- NE: Normalenvektor der Ebene E
- NS: Normalenvektor der Ebene des Strahls 35
- 36: Bedienelemente des Endoskops
- 37: Gitter
- 38, 39: Luftzutrittsöffnung
- 40, 41: Vorsprünge
- 42, 43: Abstände
- 44: Belüftungseinrichtung

## Patentansprüche

1. Vorrichtung (15) zur Zellgewinnung, Gewebebehandlung oder Gewebereinigung, insbesondere an Gewebeoberflächen (12),
mit einem Kopf (25), der einen Innenraum (26) umschließt und eine Öffnung (27) aufweist, die den Innenraum (26) mit der Umgebung verbindet und auf die Gewebeoberfläche (12) aufsetzbar ist,
mit einer Düse (31), die mit einem Speisekanal (32) verbunden und in dem Kopf (25) angeordnet sowie dazu eingerichtet ist, aus einer über den Speisekanal (32) zugeführten Flüssigkeit einen die Öffnung (27) durchquerenden Strahl (33, 35) zu erzeugen,
mit einem Absaugkanal (29), der mit dem Innenraum (26) verbunden ist
**dadurch gekennzeichnet,**
**dass** der Strahl (33, 35) schräg zu der Öffnung (27) orientiert ist.

2. Vorrichtung nach Anspruch 1, **dadurch gekennzeichnet, dass** die Öffnung (27) von einem Rand (R) umgeben ist, der eine Mündungsebene (E) festlegt, wobei der Strahl (35) mit der Flächennormalen (NE) der Mündungsebene (E) einen von Null verschiedenen spitzen Winkel (α) einschließt.

3. Vorrichtung nach einem der vorstehenden Ansprüche,
**dadurch gekennzeichnet, dass** in dem Kopf (25) der Düse (31) gegenüberliegend eine Prallfläche (34) angeordnet ist.

4. Vorrichtung nach Anspruch 3, **dadurch gekennzeichnet, dass** die Prallfläche (34) schräg zu einer von der Düse (31) vorgegebenen Richtung des Strahls (33, 35) orientiert ist.

5. Vorrichtung nach einem der vorstehenden Ansprüche, **dadurch gekennzeichnet, dass** der Kopf (25) mit einem rohrförmigen Schaft (24) verbunden ist.

6. Vorrichtung nach Anspruch 5, **dadurch gekennzeichnet, dass** der Schaft (24) dazu eingerichtet ist, in einen Arbeitskanal (20) eines Endoskops (11) eingesteckt oder auf das Ende eines Endoskops (11) aufgesteckt zu werden.

7. Vorrichtung nach einem der Ansprüche 5 oder 6, **dadurch gekennzeichnet, dass** der Schaft (24) flexibel ausgebildet ist.

8. Vorrichtung nach einem der vorstehenden Ansprüche 6 oder 7, **dadurch gekennzeichnet, dass** der Schaft (24) an dem Endoskop (11) reibschlüssig gesichert ist.

9. Vorrichtung nach einem der vorstehenden Ansprüche 5 bis 8, **dadurch gekennzeichnet, dass** die Öffnung (27) quer zu dem rohrförmigen Schaft (24) orientiert ist.

10. Vorrichtung nach einem der vorstehenden Ansprüche, **dadurch gekennzeichnet, dass** die Vorrichtung (15) einen Sitz (30) aufweist, der zur Aufnahme eines den Speisekanal (32) aufweisenden und an seinem distalen Ende mit der Düse (31) versehenen Schlauchs (19) eingerichtet ist.

11. Vorrichtung nach einem der vorstehenden Ansprüche, **dadurch gekennzeichnet, dass** der Kopf (25) eine Belüftungseinrichtung (44) aufweist, die dazu eingerichtet ist, bei auf die Gewebeoberfläche (12) aufgesetztem Kopf (25) eine Fluidverbindung zwischen der Umgebung und dem Innenraum (26) bereit zu stellen.

12. Vorrichtung nach Anspruch 11, **dadurch gekennzeichnet, dass** als Belüftungseinrichtung (44) an der Öffnung (27) wenigstens ein Abstandshalter (40, 41) angeordnet ist, der dazu eingerichtet ist, eine abdichtende Anlage der Öffnung (27) an dem Gewebe zu verhindern.

13. Vorrichtung nach Anspruch 11, **dadurch gekennzeichnet, dass** der Kopf (25) als Belüftungseinrichtung (44) zusätzlich zu der Öffnung (27) mindestens eine weitere Öffnung (38) aufweist, die zur Verbindung der Umgebung mit dem Innenraum (26) eingerichtet ist.

14. Einrichtung, bestehend aus einem Endoskop (11) mit zumindest einem Arbeitskanal (20) und einer an dem Endoskop (11) befestigten Vorrichtung (15) nach einem der vorstehenden Ansprüche.

15. Verfahren zur Beprobung von Gewebeoberflächen mittels einer Vorrichtung nach Anspruch 1, wobei bei dem Verfahren:
ein Strahl (35) aus Flüssigkeit unter einem spitzen Winkel auf eine Gewebeoberfläche (12) gerichtet wird,
so dass wenigstens einige Zellen von der Gewebeoberfläche (12) gelöst werden, und
von sich von der Gewebeoberfläche (12) lösende Flüssigkeitstropfen aufgenommen und mit einem Luft- oder
Gasfluss durch den Absaugkanal (29) zu einer Sammeleinrichtung (16) geführt werden.
